# EUROPEAN PATENT SPECIFICATION

(11) **EP 2 131 756 B1**
(45) Date of publication and mention of the grant of the patent: **19.09.2018**
(21) Application number: 08732941.3
(22) Date of filing: 27.03.2008
(51) Int. Cl.: A61B 17/12, A61B 1/00

(54) **ENDOSCOPIC SECURING SYSTEM**
ENDOSKOPISCHES FIXIERSYSTEM
SYSTÈME DE FIXATION ENDOSCOPIQUE

(30) Priority: 30.03.2007 US 920855 P
(43) Date of publication of application: 16.12.2009
(73) Proprietor: Cook Medical Technologies LLC, Bloomington, IN 47404 (US)
(72) Inventor: CARTER, Matthew, P., Dobson, NC 27017 (US)
(74) Representative: Garratt, Peter Douglas
(86) International application number: PCT/US2008/058471
(87) International publication number: WO 2008/121722

(56) References cited:
- WO-A-2004/021865
- WO-A-2008/048408
- US-A- 6 042 591
- US-A1- 2006 052 662
- US-A1- 2006 258 906

## Description

### CROSS REFERENCE TO RELATED APPLICATION

This application claims priority to United States Provisional Application No. 60/920,855, filed on March 30, 2007.

### TECHNICAL FIELD

The present invention relates to an improved endoscopic securing system having safety features to secure a cap member to the distal end of an endoscope and prevent the dislodgement of a cap member from an endoscope.

### BACKGROUND

The treatment of tissue encompasses a variety of techniques such as electrocauterization, heat therapy, resection (removal of tissue), and sclerotherapy (the injection of medicine into target tissue). These treatment techniques usually involve the passing of medical instruments through the operating channel of the endoscope. The endoscope permits minimally invasive access, as well as visualization and suction aids.

Another technique that frequently utilizes the operating channel of the endoscope is ligation, which involves applying a band or ligature around a vessel or portion of tissue, thereby cutting off blood or fluid flow and causing the tissue to necrose and separate from adjacent healthy tissue. Ligation is widely used to treat a number of medical tissue conditions, including, but not limited to, hemorrhoids, polyps, ballooning varices, and other types of lesions, including those that are cancerous. Typically, ligators are also used with a suction or vacuum means to draw the tissue into the distal end, whereby the band is deployed over the base of the diseased tissue to cut off blood flow. The ligating device is typically activated by retracting a line (string, wire, or cable) that is attached to the ligator at the distal end of an endoscope and is threaded through the operating channel of the endoscope to the proximal end of the instrument. The ligator can be activated by mechanically pulling the activating line by means of a hand-operated reel or trigger, or by a motor drive mechanism. Various other ligating devices use cooperating inner and outer members that slide the individual bands by pushing or pulling them from the end of the inner or outer member, the bands being preloaded onto the inner or outer member prior to deployment.

A ligator device can become dislodged during an endoscopic procedure. The ligator is typically placed on the end of an endoscope covering the cap. Due to the sometimes vigorous movements of the endoscope, the ligator can become dislodged and separated from the endoscope. To recover the ligator, a physician will typically have to withdraw the endoscope and then re-enter with a grasping device, such as forceps, to retrieve the ligator. This retrieval delays the surgical procedure and places the patient at greater risk. Therefore, there is a need for improved ligator devices with safety features that prevent their dislodgement and/or separation from an endoscope cap. There is also a need for a means of efficiently retrieving a device that has become dislodged and/or separated from the endoscope.

Reference is directed to US 6,042,591 and WO2004/0218665 which both disclose a ligating band dispenser having an actuating mechanism which can include one or more tethers.

### BRIEF SUMMARY

The present invention provides an endoscopic securing system according to claim 1 for devices used in the endoscopic treatment of tissue. The system comprises a cap member configured to be disposed on the distal end of an endoscope, a safety harness, and a safety coupling. The safety coupling is adapted to be securable to the endoscope between the proximal end of the endoscope and the cap member. The safety coupling is formed to fit around the endoscope and the safety harness is connected to the cap member and extends in a proximal direction to connect with the safety coupling. In one embodiment of the present invention, the securing system further comprises an endoscope with a ridge distally adjacent to the safety coupling that prevents the safety coupling from moving distally toward the distal end of the endoscope. According to the invention, the safety harness comprises one of at least one tether.

The cap member can be a ligating barrel, sectomy hood, or any other endoscopic device that can be attached to the distal end of an endoscope. If a ligating barrel is used, there can be one or more ligating bands removably disposed on the barrel. In other embodiments of the invention, there can be two tethers connecting the cap member and the safety coupling. In examples not covered by the scope of the patent, the tethers can extend through the safety coupling to a point near the proximate end of the endoscope so that they can be coupled to an activation device.

The endoscopic securing system secures the cap member to the distal end of an endoscope. The method comprises providing a cap member disposed on the distal end of an endoscope, a safety harness connected to the cap member and extending in a proximal direction from the cap member, and coupled to a safety coupling. The safety coupling is secured to the endoscope at a location proximal to and apart from the cap member. Then the safety harness is extended from the cap member in a proximal direction approaching the safety coupling.

According to examples not covered by the scope of the patent, the endoscopic securing system is also well suited for use in methods of retrieving a dislodged cap member by using an actuating device to pull the tether proximally. The cap member can be retrieved by withdrawing the endoscope in some embodiments.

### BRIEF DESCRIPTION OF THE DRAWINGS

Figure 1 is a perspective view of an endoscopic securing system.
Figure 2 is a perspective view of the endoscopic securing system disposed on an endoscope.
Figure 3 presents a side view and a cross sectional view of a safety coupling. Figure 3A is a cross sectional view of a safety coupling
Figure 4 is a cross sectional view showing auxiliary access channels for auxiliary endoscopic instruments.
Figure 5 is a perspective view of a securing sleeve disposed on an endoscope.

### DETAILED DESCRIPTION OF THE DRAWINGS AND THE PRESENTLY PREFERRED EMBODIMENTS

The present invention provides a securing system for use with cap members adapted to be disposed on the distal end of an endoscope. At least one tether extends from the cap member and along the endoscope to a safety coupling. The safety harness is disposed around the endoscope at a location between the proximal end of the endoscope and the cap member. This location can vary depending on the length and type of safety harness used. A ridge can be disposed on the endoscope that prevents the safety coupling from sliding toward the distal end of the endoscope. The safety harness secures the cap member on the distal end of the endoscope and can be used to retrieve a cap member that becomes dislodged from the distal end of the endoscope. The safety harness is characteristically taut to assist in securing and stabilizing the cap member on the distal end of the endoscope. The safety coupling has a fastening mechanism such that it has a secure fit around the shaft of the endoscope thereby preventing the coupling from sliding distally. The inner surface of the safety coupling can be textured or comprise an adhesive or other means to provide frictional engagement with the outer surface of the endoscope.

Examples described herein do not fall within the scope of the patent. In some examples, the most proximal end of the safety harness never enters the patient such that an operator may actuate the safety harness to retrieve the cap member. According to the invention, the proximal end of the safety harness terminates at the safety coupling and typically enters the GI tract. The cap member can comprise any removable device attached to the scope to help provide a particular diagnostic or therapeutic function. The cap member can be, for example, a ligating barrel, endoscope hood, or other device that attaches to the distal end of an endoscope. Embodiments that may be preferred comprise cap members adapted to receive the proximal end of the safety harness. However, the safety harness of the present invention may be secured to current cap members known in the art.

Because the cap member is attached to the safety coupling by a safety harness, the likelihood that the cap member becomes dislodged is greatly decreased. In instances where the cap member becomes dislodged from the distal end of the endoscope, the securing system prevents the cap member from becoming lost in the body. In other embodiments, however, the safety harness is sufficiently flaccid to allow the cap member to move freely as the endoscope is maneuvered throughout the body. The safety harness can be at least one tether, elastic member (band, cord, or the like), spring, or a securing sleeve. The safety harness can be made of a resilient material that places tension on the cap member when the safety coupling is appropriately spaced. The tether can be made of synthetic fiber, string, wire, cable, or other suitable biocompatible material. The tether can be connected to any point on the safety coupling that provides secure attachment. In other examples, the tether proceeds through safety coupling channels to an actuation device. The length of the tether from the cap member to the safety coupling can be predetermined based on the procedure and patient. In some embodiments the length of the tether(s) can be from about 2 cm to about 20 cm or any length therein. The length of the tether(s) from the safety coupling to the proximal end of the endoscope can vary depending on the procedure and the particular scope used. The tether lengths from the safety coupling to the proximal end can be from about 100 cm to about 200 cm or any length therein. Such tether length(s) will be long enough to extend from the safety coupling to the proximal end of the endoscope and, in some embodiments, long enough to attach to an actuation device.

The securing sleeve is connected to the cap member and covers a portion of the endoscope from the cap member and extends in a proximal direction over the endoscope. The securing sleeve is generally tubular in shape and is comprised of material suitable for providing frictional engagement with the endoscope. The securing sleeve is deployable over the body of the endoscope to provide a friction fit with the endoscope. In some embodiments the securing sleeve is deployable by unfurling or unrolling the sleeve over the body of the endoscope in a proximal direction away from the cap member. As such, the examples also provide an endoscopic securing system comprising a cap member, as described above, connected to a tubular safety harness, such as a securing sleeve, to prevent dislodgment of the cap member extending in a proximal direction from the cap member. In such examples the safety coupling may or may not be used.

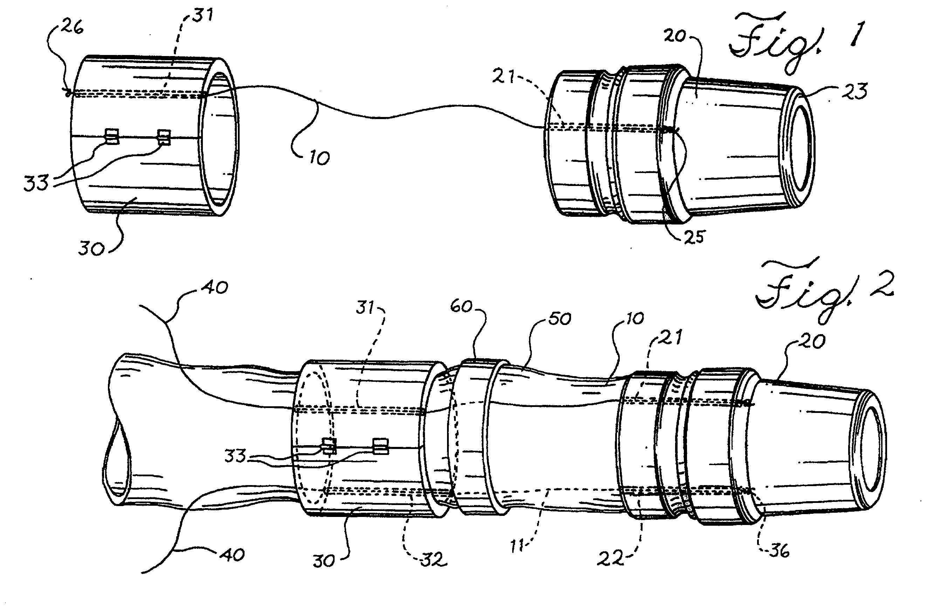

In some examples, the tether **10** can be movably attached to the safety coupling **30** while terminating in a cap member **20.** As shown in Figure 1, the tether 10 is connected to the cap member **20** by threading the tether **10** through a first channel **21** and tying a knot **25** at the distal opening **23** of the first channel **21** to prevent the tether **10** from decoupling. Although the tether **10** is connected to the safety coupling **30** and cap member **20,** in this example by knots, it is understood that the tether **10** can be connected by other means sufficient to secure attachment of the tether **10** to the safety coupling **30** and/or cap member **20.** According to the invention, the tether may be attachable by cinching or clamping. According to some examples, the tether **10** can also be coupled to the safety coupling **30** by threading the line **10** through a first safety coupling channel **31** and tying a knot **26** at the proximal end of the channel **31.** If the cap member **20** is dislodged from the distal end of the endoscope **50,** a surgeon can recover the cap member **20** by withdrawing the endoscope **50** and reattaching the cap member **20** onto the distal end of the endoscope **50.** Once reattached, the surgeon can reinsert the cap member **20** without a significant loss of operating time. There are also embodiments comprising one tether **10** or, such as in Figure 2, at least two tethers **10** and **11** extending from the cap member **20** and terminating at the safety coupling **30.**

In the example shown in Figure 2, a second tether **11** is threaded through a second cap member channel **22** and subsequently coupled by a knot **36** tied at the distal end of the second cap member channel **22.** The second tether **11** extends axially along the endoscope **50** to a second safety coupling channel **32.** The tethers **10** and **11,** as shown in Figure 2, are tightly drawn to secure the cap member **20** to the distal end of the endoscope **50.** This decreases the likelihood of dislodgment. In some examples, the proximate end of the second tether **11** can be coupled to the safety coupling **30** by way of a knot (not shown), or the like, in the proximate end of the first **31** and second **32** safety coupling channels. Distally adjacent to the safety coupling **30** is a ridge **60** on the endoscope **50** that prevents the safety coupling **30** from sliding distally in the direction of the cap member **20.** This ridge **60** is present on many types of endoscopes **50** and is formed by the proximal end of a flexible sheath that is disposed over the distal most portion of the endoscope **50.** A dislodged cap member in this embodiment can be retrieved and reattached as previously discussed.

Figure 2 shows an example where the tethers **10** and **11** extend from the cap member **20** through the safety coupling **30** without being affixed thereto. According to the invention, the tethers **10** and **11** are affixed or otherwise coupled to the safety coupling **30** but do not extend through channels **31** and **32** in the safety coupling. According to some examples, the proximate ends **40** of the tethers **10** and **11** preferably terminate at an actuation device at or near the proximate end of the endoscope **50.** The proximate ends **40** can be attached to an actuation device such as a hand operated reel, trigger, or motor driven mechanism. The tethers **10** and **11** can also be actuated using the hand of a surgeon. Some examples have one tether or more than two tethers extending through the safety coupling **30** to the proximate end of the endoscope. Although shown with safety channels **10** and **11** in this example, multiple safety channels can be used with accompanying tethers.

In examples where the tethers extend to the proximate end of the endoscope **50,** the proximate ends **40** of the tethers can be manipulated by the operator in the event the cap member **20** becomes dislodged from the distal end of the endoscope. Also, in such examples, there can be one or more proximate ends **40** extending from the safety coupling **30** to the proximate end of the endoscope **50.** The proximate ends **40** can be attached to an actuation device so that an operator can pull the proximate ends **40** of the tethers **10** and **11** to retrieve the cap member **20** in the case of dislodgement. In such an event, the operator pulls proximally on the proximate ends **40** to move the safety coupling **30** in a proximal direction. By doing so, the cap member **20** is also moved in a proximal direction. The operator can then retrieve the cap member **20** by withdrawing the endoscope **50** fully from the body. In some embodiments, the operator can be capable of reattaching the cap member **20** on the distal end of the endoscope without withdrawing the endoscope from the body.

Cap members such as ligating barrels are suitable for this invention and are known in the art. A more detailed description of such barrels can be found in U.S. Patent No. 5,624,453, incorporated herein by reference in its entirety. The ligating barrels can have one or more ligating bands removably disposed on the barrel. Endoscope hoods are also suitable cap members useful in some embodiments of the present invention. The securing system of the present invention can be used with any cap member adapted to fit on the distal end of an endoscope and used in endoscopic procedures.

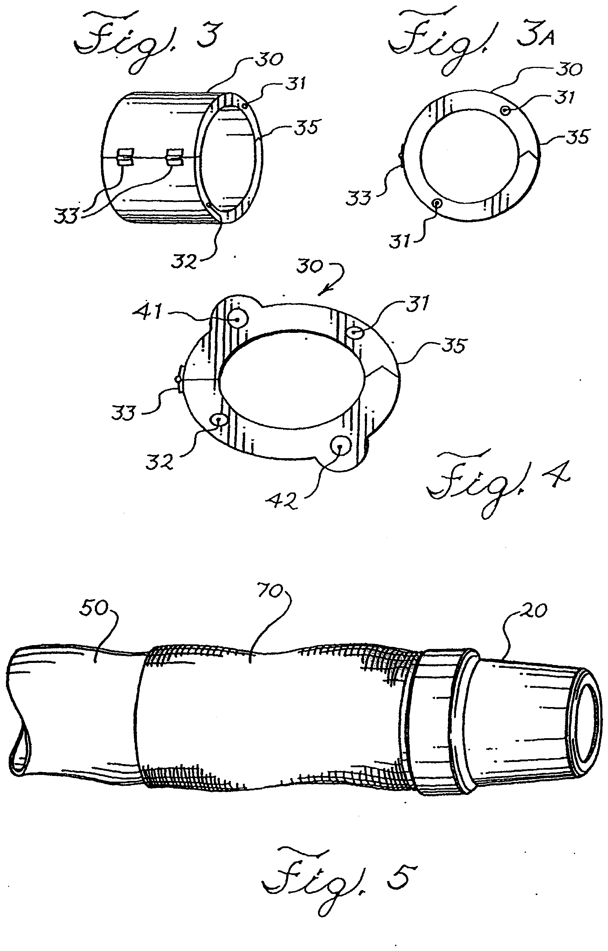

The safety coupling **30** can be made of a resilient material such as thermoplastics, polyurethanes, polycarbonates, or other polymers or metals. As shown in Figure 3, the safety coupling **30** resembles a ring or a circular clamp that is attached to the endoscope with a fastening mechanism such as a clasp, buckle, friction fit, strap, or a snap fit **35,** with a projection and a recess. The safety coupling is formed to fit around the body of an endoscope. Hinges **33** are located on the opposite side of the safety coupling **30** shown in Figures 2, 3, 3A, and 4. The safety coupling **30** may have one, two, or more hinges. There also may be embodiments without hinges having a jointed device or flexible piece on which the sides of the safety coupling **30** moves. The safety coupling **30** can be from about 0.5 cm to about 3 cm in length or any combination or subcombination thereof. The overall size of the clamp and endoscope is dependent on the size of the patient's GI tract. The safety coupling is sized to smoothly travail the tract without being impeded.

Another embodiment of the safety system provides a safety coupling **30** as shown in Figure 4 that comprises channels **31** and **32** in the walls of the safety coupling and auxiliary access channels **41** and **42** on the periphery of the safety coupling **30.** The latter being used to accommodate auxiliary endoscopic instruments to be advanced therethrough. Such instruments include forceps, snares, or any other instrument used to contact the body cavity for performing biopsy or other similar functions. In the embodiment illustrated in Figure 4, a portion of the wall has been thickened to accommodate the larger diameter auxiliary access channels **41** and **42.** There are also embodiments comprising at least one auxiliary access channel or at least three.

The safety coupling **30** is adapted to fit snugly around the circumference of an endoscope **50.** The inner surface of the safety coupling **30** can comprise an elastomeric material to increase friction between the endoscope **50** and the safety coupling **30.** The inner surface of the safety coupling **30** can be internally ridged or textured to assist in preventing movement of the safety coupling **30.** The safety coupling **30** has a diameter slightly larger than the diameter of the endoscope **50** body sufficient to provide a secure fit and to prevent the clamp **30** from sliding. In other embodiments, the safety coupling diameter is slightly smaller than the diameter of the ridge **60** positioned distal to the safety coupling **30** such that the ridge **60** prevents distal movement of the safety coupling **30.** The ridge **60** can be integrated into an endoscope **50** and/or specifically made for use with the securing systems described herein. For the securing systems used on other endoscopes, the safety coupling **30** fit can be secure enough to prevent distal movement. To further prevent distal movement, one or more elastomeric bands can be placed along the length of the endoscope to maintain the tethers.

As depicted in Figure 5, the safety harness according to an example can be a securing sleeve **70.** The securing system may comprise a securing sleeve **70** that extends from the cap member **20** in a proximal direction. Such an example is installed by unrolling the tubular securing sleeve **70** over the distal end of the endoscope **50.** The sleeve 70, preferably, and in some examples, the securing tether, has an inner surface that provides frictional engagement with the endoscope. Such examples may or may not have a clamp as the friction fit is sufficient to hold the cap member **20** in place. The securing sleeve **70** or tether can made of an elastomeric material, for example, polyurethane-based elastomer, polyester-based elastomer, polyolefine-based elastomer, polyamide-based elastomer, polystyrene-based elastomer, fluorine-based elastomer, silicone rubber, fluororubber, latex rubber, and the like. They can be used alone or as a mixture of two or more thereof.

An alternate means of securing the cap member includes a helical securing tether configured for attachment to an endoscope. The distal end of the securing tether is attached to the cap member and has a fixed diameter. The diameter is adjustable in a proximal direction along the tether. The helical securing tether wraps around the endoscope and conforms to the endoscope's outer diameter with sufficient gripping force to secure the cap member thereto. The securing tether forms a spiral configured to wind about an endoscope with frictional engagement. When the securing tether is wrapped around an endoscope having a diameter that approximates the diameter of the distal end of the tether, the number of turns decreases. The number of turns increases when the securing tether adjusts to fit around an endoscope having a diameter that is less than the diameter of the distal end of the securing tether. This securing tether can be comprised of an elastomeric polymer or wire.

Generally, attachment of the endoscopic securing system to an endoscope is a method of securing a cap member to the distal end of an endoscope. One method comprises the steps of attaching a cap member to the distal end of the endoscope; providing a safety coupling attaching the safety coupling to an endoscope; and coupling the safety coupling to the cap member using at least one safety line. Another embodiment comprises providing a cap member disposed on the distal end of an endoscope, at least one safety line, and a safety coupling. The safety coupling is secured to the endoscope at a location proximal to and apart from the cap member. At least one tether is coupled between the cap member and the safety coupling. According to some examples, this coupling can be done by threading the line through a channel in the cap member and securing the line in the channel or at the proximal opening of the channel by a knot. Other ways to couple the tether to the cap member include by adhesive or molded into the safety coupling or cap member. The safety coupling is attached to the endoscope as well as the cap member. In some embodiments, there is a ridge distally adjacent to the safety coupling that prevents the safety coupling from moving distally toward the cap member. In other embodiments the cap member is disposed on the distal end of the endoscope and the safety coupling is disposed about the endoscope proximal of and apart from the cap member.

Also provided herein are methods of retrieving a cap member that becomes dislodged from an endoscope using the endoscopic securing systems of the present invention. When a cap member that is part of the present system becomes separated from an endoscope, the cap member remains in contact with the safety coupling by way of at least one safety line. In embodiments where the tethers terminate and connect to the safety coupling, an operator can pull the endoscope out of the body to retrieve the cap member. This eliminates the need to use forceps to retrieve the device. In those examples where the tethers extend through the safety coupling to an actuation device, the cap member can be pulled proximally by actuation of the tethers to prevent the cap member from becoming lost in the body. In some instances, an operator can be capable of re-securing the cap member onto the endoscope.

It is therefore intended that the foregoing detailed description be regarded as illustrative rather than limiting, and that it be understood that it is the following claims that are intended to define the scope of this invention.

## Claims

1. An endoscopic securing system comprising an endoscope, a cap member (20) taking the form of an endoscopic device disposed on the distal end of the endoscope, a safety harness (10), and a safety coupling (30) secured to the endoscope between a proximal end of the endoscope and the cap member at a location proximal to and apart from the cap member, the safety coupling formed to fit around the endoscope and having a fastening mechanism preventing the safety coupling from sliding distally, and the safety harness connected to the cap member and extending in a proximal direction to connect with the safety coupling to reduce the likelihood of the cap member becoming dislodged from the distal end of the endoscope; wherein the safety harness comprises at least one tether affixed to the safety coupling and wherein the or each tether does not extend through the safety coupling.

2. The system of claim 1 wherein the cap member is a ligating barrel or endoscope hood.

3. The system of claim 1 further comprising an endoscope with a ridge spaced apart from a distal end thereof, wherein the safety coupling is disposed about the endoscope proximal of the ridge so as to prevent the safety coupling from moving distally toward a distal end of the endoscope.

4. The system of claim 1 wherein with the cap member dislodged from the distal end of the endoscope, the safety harness and safety coupling prevent the cap member from being lost in the body.

5. The system of claim 1 wherein the safety coupling further comprises at least one auxiliary access channel on the periphery of the safety coupling to accommodate forceps or other instruments to contact the body cavity.

6. The system of claim 5 wherein the cap member is a ligating barrel and the system further comprises one or more ligating bands removably disposed on the ligating barrel.

7. The system of claim 1 comprising at least two tethers.

8. A method of securing a cap member to an endoscope comprising:
providing the endoscopic securing system of claim 1;
securing the safety coupling to the endoscope at a location proximal to and apart from the cap member; and
extending the safety harness from the cap member in a proximal direction approaching the safety coupling.

## Patentansprüche

1. Endoskopisches Fixiersystem umfassend ein Endoskop, ein Kappenelement (20) in Form einer endoskopischen Vorrichtung, die am distalen Ende des Endoskops angeordnet ist, einen Sicherheitsgurt (10) und eine Sicherheitskupplung (30), die am Endoskop zwischen einem proximalen Ende des Endoskops und dem Kappenelement an einer Stelle proximal zum und entfernt vom Kappenelement befestigt ist, wobei die Sicherheitskupplung so ausgebildet ist, dass sie um das Endoskop passt, und einen Befestigungsmechanismus aufweist, der distales Gleiten der Sicherheitskupplung verhindert, und wobei der Sicherheitsgurt mit dem Kappenelement verbunden ist und sich in einer proximalen Richtung zur Verbindung mit der Sicherheitskupplung erstreckt, um die Wahrscheinlichkeit zu verringern, dass das Kappenelement vom distalen Ende des Endoskops verdrängt wird; wobei der Sicherheitsgurt mindestens ein Spannseil umfasst, das an der Sicherheitskupplung befestigt ist und wobei das oder jedes Spannseil sich nicht durch die Sicherheitskupplung erstreckt.

2. System nach Anspruch 1, wobei das Kappenelement ein Ligaturzylinder oder eine Endoskophaube ist.

3. System nach Anspruch 1, ferner umfassend ein Endoskop mit einer Leiste, die von einem distalen Ende davon beabstandet ist, wobei die Sicherheitskupplung um das Endoskop proximal von der Leiste entfernt ist, um zu verhindern, dass sich die Sicherheitskupplung distal zum einem distalen Ende des Endoskops bewegt.

4. System nach Anspruch 1, wobei der Sicherheitsgurt und die Sicherheitskupplung verhindern, dass das Kappenelement im Körper verloren geht, wenn das Kappenelement vom distalen Ende des Endoskops verdrängt wurde.

5. System nach Anspruch 1, wobei die Sicherheitskupplung ferner mindestens einen Hilfszugangskanal auf dem Umfang der Sicherheitskupplung umfasst, um Zangen oder andere Instrumente zum Kontaktieren der Körperhöhle aufzunehmen.

6. System nach Anspruch 5, wobei das Kappenelement ein Ligaturzylinder ist und das System ferner ein oder mehrere Ligaturbänder umfasst, die entfernbar auf dem Ligaturzylinder angeordnet sind.

7. System nach Anspruch 1, umfassend mindestens zwei Spannseile.

8. Verfahren zur Befestigung eines Kappenelements an einem Endoskop, umfassend:
Bereitstellen des endoskopischen Fixiersystems nach Anspruch 1;
Fixieren der Sicherheitskupplung am Endoskop an einer Stelle proximal zum und entfernt vom Kappenelement; und
Strecken des Sicherheitsgurts vom Kappenelement in einer proximalen Richtung, sich der Sicherheitskupplung annähernd.

## Revendications

1. Système de fixation endoscopique comprenant un endoscope, un élément formant capuchon (20) prenant la forme d'un dispositif endoscopique disposé sur l'extrémité distale de l'endoscope, un dispositif de liaison de sécurité (10) et un organe d'accouplement de sécurité (30) fixé à l'endoscope entre une extrémité proximale de l'endoscope et l'élément formant capuchon au niveau d'un emplacement en position proximale par rapport à l'élément formant capuchon et espacé de celui-ci, l'organe d'accouplement de sécurité étant formé de façon à pouvoir s'adapter autour de l'endoscope et comportant un mécanisme d'attache empêchant l'organe d'accouplement de sécurité de glisser dans la direction distale, et le dispositif de liaison de sécurité étant raccordé à l'élément formant capuchon et s'étendant dans une direction proximale afin de se raccorder à l'organe d'accouplement de sécurité de façon à réduire la probabilité que l'élément formant capuchon ne soit délogé de l'extrémité distale de l'endoscope ; dans lequel le dispositif de liaison de sécurité comprend au moins un organe d'assujettissement fixé à l'organe d'accouplement de sécurité et dans lequel le ou chacun des organes d'assujettissement ne s'étend pas à travers l'organe d'accouplement de sécurité.

2. Système selon la revendication 1, dans lequel l'élément formant capuchon est un cylindre de ligature ou une coiffe d'endoscope.

3. Système selon la revendication 1, comprenant en outre un endoscope comportant une nervure espacée de son extrémité distale, dans lequel l'organe d'accouplement de sécurité est disposé autour de l'endoscope en position proximale par rapport à la nervure de façon à empêcher l'organe d'accouplement de sécurité de se déplacer dans la direction distale vers une extrémité distale de l'endoscope.

4. Système selon la revendication 1, dans lequel, lorsque l'élément formant capuchon est délogé de l'extrémité distale de l'endoscope, le dispositif de liaison de sécurité et l'organe d'accouplement de sécurité empêchent l'élément formant capuchon d'être perdu dans le corps.

5. Système selon la revendication 1, dans lequel l'organe d'accouplement de sécurité comprend en outre au moins un canal d'accès auxiliaire sur la périphérie de l'organe d'accouplement de sécurité destiné à recevoir des forceps ou d'autres instruments destinés à venir en contact avec la cavité corporelle.

6. Système selon la revendication 5, dans lequel l'élément formant capuchon est un cylindre de ligature et le système comprenant en outre un ou plusieurs anneaux de ligature disposés de manière amovible sur le cylindre de ligature.

7. Système selon la revendication 1 comprenant au moins deux organes d'assujettissement.

8. Procédé de fixation d'un élément formant capuchon sur un endoscope comprenant :
prendre le système de fixation endoscopique selon la revendication 1 ;
fixer l'organe d'accouplement de sécurité à l'endoscope au niveau d'un emplacement en position proximale par rapport à l'élément formant capuchon et espacé de celui-ci ; et
étendre le dispositif de liaison de sécurité à partir de l'élément formant capuchon dans une direction proximale allant vers l'organe d'accouplement de sécurité.
